# EUROPEAN PATENT APPLICATION

(11) **EP 3 782 532 A1**
(43) Date of publication of application: **24.02.2021**
(21) Application number: 19788708.6
(22) Date of filing: 04.03.2019
(51) Int. Cl.: A61B 1/07, G02B 23/26

(54) **MEDICAL SYSTEM, CONNECTION STRUCTURE, AND CONNECTION METHOD**

(30) Priority: 18.04.2018 JP 2018079587
(71) Applicant: Sony Corporation, 108-0075 Tokyo (JP)
(72) Inventor: NAKANO, Tomu, Tokyo 108-0075 (JP); KATOU, Syougo, Tokyo 108-0075 (JP)
(74) Representative: D Young & Co LLP
(86) International application number: PCT/JP2019/008359
(87) International publication number: WO 2019/202860

(57) **Abstract**

To allow illumination light emitted from a light source device to optimally enter a light guide cable in accordance with a diameter of a light guide portion of the light guide cable.

Provided is a medical system including medical equipment that includes an imaging unit that images an observation target, and the light source device that emits light for illuminating the observation target and illuminates the observation target with the light via the light guide cable, in which an entrance end face of the light guide cable is set to a different position with respect to a focal plane of light emitted from the light source device in accordance with the diameter of the light guide portion of the light guide cable.

## Description

### TECHNICAL FIELD

The present disclosure relates to a medical system, a connection structure, and a connection method.

### BACKGROUND ART

Conventionally, for example, Patent Document 1 below describes that at least one laser light source is included and light from the laser light source enters a light guide.

### CITATION LIST

### PATENT DOCUMENT

Patent Document 1: Japanese Patent Application Laid-Open No. 2015-223462

### SUMMARY OF THE INVENTION

### PROBLEMS TO BE SOLVED BY THE INVENTION

For example, as a light guide cable used in a medical system, light guide cables that differ in light guide portion diameter may be used. For example, in a case of a surgical space large enough to use an endoscope with a larger diameter or a larger microscope, there is a larger space for storing a light guide cable, so a thicker light guide cable having a larger-diameter light guide portion that allows more illumination light to enter is used.

On the other hand, in a case where the surgical space is so small that a thinner endoscope or a smaller microscope needs to be used, the space for storing a light guide cable is smaller, so a thinner light guide cable having a smaller-diameter light guide portion that allows less illumination light to enter but can be stored in a smaller space is used.

Light emitted from a light source device is refracted by a lens and formed as an image on a focal plane. In a case where the diameter of the light guide portion is substantially equal to a size of illumination light formed as an image on the focal plane, an entrance end face of the light guide cable is arranged at a position of the focal plane so that illumination light enters the light guide cable most and uniformly. However, in a case of a thicker light guide cable with a diameter larger than the size of illumination light formed as an image on the focal plane, arranging the entrance end face at the focal plane causes a problem in that it is not possible to sufficiently secure uniformity of brightness of an observation target illuminated with light by the light guide cable.

As conventional medical light sources, lamp light sources (xenon lamps and halogen lamps), white LEDs, and the like are mainly used. In a case where these light sources are used, the size of illumination light on the focal plane is larger than the diameter of the light guide portion of the light guide cable, both a thicker light guide cable having a larger-diameter light guide portion and a thinner light guide cable having a smaller-diameter light guide portion allow illumination light to enter the light guide cable most and uniformly. Thus, a relative positional relationship between the focal plane and the entrance end face of the light guide cable has not been taken into consideration in accordance with the diameter of the light guide portion, and the position of the light guard cable in an optical axis direction with respect to the focal plane has been the same regardless of the diameter of the light guide portion.

However, in a case where a laser having a high light-collecting property is used as a light source, the size of illumination light on the focal plane becomes smaller than the diameter of the light guide portion of the light guide cable. In this case, the fixed position of the cable end face where illumination light enters most and uniformly differs between a thicker light guide cable having a larger-diameter light guide portion and a thinner light guide cable having a smaller-diameter light guide portion.

It has therefore been required to allow illumination light emitted from the light source device to optimally enter the light guide cable in accordance with the diameter of the light guide portion of the light guide cable.

### SOLUTIONS TO PROBLEMS

The present disclosure provides a medical system including medical equipment that includes an imaging unit that images an observation target, and a light source device that emits light for illuminating the observation target and illuminates the observation target with the light via a light guide cable, in which an entrance end face of the light guide cable is set to a different position with respect to a focal plane of light emitted from the light source device in accordance with a diameter of a light guide portion of the light guide cable.

Furthermore, the present disclosure provides a connection structure between a light source device and a light guide cable, in which an entrance end face of the light guide cable is set to a different position with respect to a focal plane of light emitted from the light source device in accordance with a diameter of a light guide portion of the light guide cable.

Furthermore, the present disclosure provides a connection method of connecting a light source device and a light guide cable, the connection method including positioning a light entrance end face of the light guide cable at a different position with respect to a focal plane of light emitted from the light source device in accordance with a diameter of a light guide portion of the light guide cable.

### EFFECTS OF THE INVENTION

As described above, according to the present disclosure, it is possible to allow illumination light emitted from the light source device to optimally enter the light guide cable in accordance with the diameter of the light guide portion of the light guide cable.

Note that the effects described above are not necessarily restrictive. In addition to or in place of the effects described above, any of the effects described in the present specification or other effects that can be grasped from the present specification may be exerted.

### BRIEF DESCRIPTION OF DRAWINGS

Fig. 1 is a schematic diagram illustrating a light source device and a light guide cable connected to the light source device according to an embodiment of the present disclosure.
Fig. 2 is a cross-sectional view illustrating in detail a connection portion of the light source device.
Fig. 3 is a schematic diagram illustrating a state in which two types of light guide cables that differ in light guide portion thickness are each attached to the connection portion of the light source device.
Fig. 4 is a schematic diagram illustrating a state in which the two types of light guide cables that differ in light guide portion thickness are each attached to the connection portion of the light source device.
Fig. 5 is a schematic diagram illustrating a configuration in which an insertion portion of the connection portion has a tapered surface.
Fig. 6 is a cross-sectional view illustrating in detail a state in which the connection portion of the light source device illustrated in Fig. 5 is connected with a connection portion of the light guide cable.
Fig. 7 is a cross-sectional view illustrating in detail a state in which the connection portion of the light source device illustrated in Fig. 5 is connected with the connection portion of the light guide cable.
Fig. 8 is a cross-sectional view illustrating an example in which an inner surface of the insertion portion of the connection portion in the configuration illustrated in Fig. 2 is stepped only in a part of an inner diameter.
Fig. 9 is a cross-sectional view illustrating in detail a state in which the connection portion of the light source device illustrated in Fig. 8 is connected with the connection portion of the light guide cable.
Fig. 10 is a cross-sectional view illustrating in detail a state in which the connection portion of the light source device illustrated in Fig. 8 is connected with the connection portion of the light guide cable.
Fig. 11 is a schematic diagram for illustrating a configuration in which a position of the connection portion in an optical axis direction is changed in accordance with brightness of an observation target illuminated by the light guide cable.
Fig. 12 is a schematic diagram for illustrating a configuration in which the position of the connection portion in the optical axis direction is changed in accordance with brightness of an observation target illuminated by the light guide cable.
Fig. 13 is a diagram illustrating an example of a schematic configuration of an endoscopic surgery system.
Fig. 14 is a block diagram illustrating an example of a functional configuration of a camera head and a CCU illustrated in Fig. 13.
Fig. 15 is a diagram illustrating an example of a schematic configuration of a microscopic surgery system to which the light source device according to the present disclosure can be applied.

### MODE FOR CARRYING OUT THE INVENTION

A preferred embodiment of the present disclosure will be described below in detail with reference to the accompanying drawings. Note that, in the present specification and drawings, components having substantially the same functional configurations are denoted by the same reference numerals, and the description thereof will thus not be repeated.

Note that the description will be given in the order below.
1. Overall configuration including light source device and light guide cable
2. Connection structure between light source device and light guide cable
3. Variations of present embodiment
3.1. Example in which insertion portion has tapered surface
3.2. Example in which inner surface of insertion portion is stepped only in part of inner diameter
3.3. Configuration example in which position of entrance end face can be changed by actuator
4. Configuration example of medical system
4.1. Configuration example of endoscope system
4.2. Configuration example of microscope system

### 1. Overall configuration including light source device and light guide cable

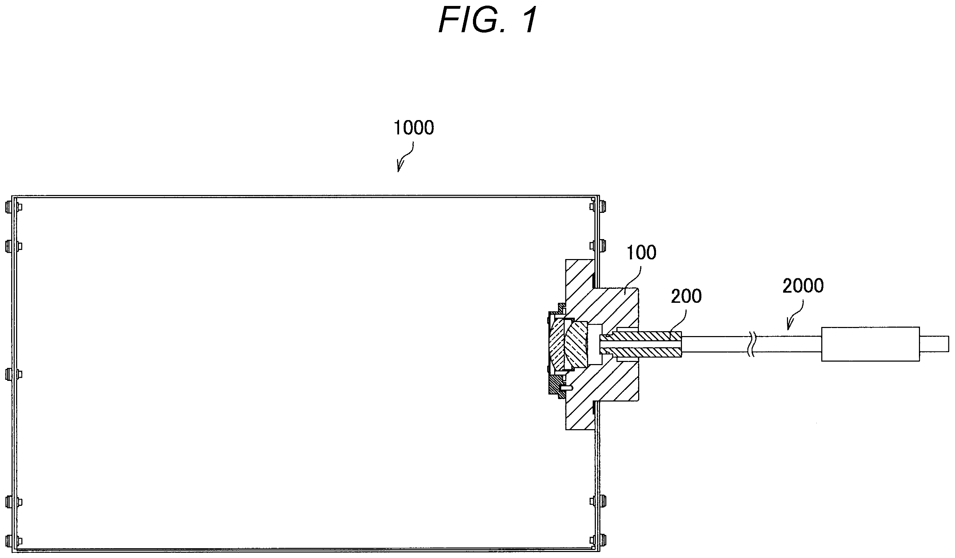

Fig. 1 is a schematic diagram illustrating a light source device 1000 and a light guide cable 2000 connected to the light source device 1000 according to an embodiment of the present disclosure. The light source device 1000 and the light guide cable 2000 are applied to, for example, a medical system. The light source device 1000 generates light such as laser light as an example, and emits the laser light to the light guide cable 2000. The light guide cable 2000 guides light emitted from the light source device 1000, and illuminates an observation target with the light.

A connection portion 100 is provided at a light emitting portion of the light source device 1000. Furthermore, the light guide cable 2000 is provided with, at an end thereof, a connection portion 200 where light emitted from the light source device 1000 enters. Fig. 1 illustrates a cross section passing through a central axis (optical axis) of the connection portion 100 and the connection portion 200. The connection portion 100 and the connection portion 200 constitute a connection structure according to the present disclosure.

### 2. Connection structure between light source device and light guide cable

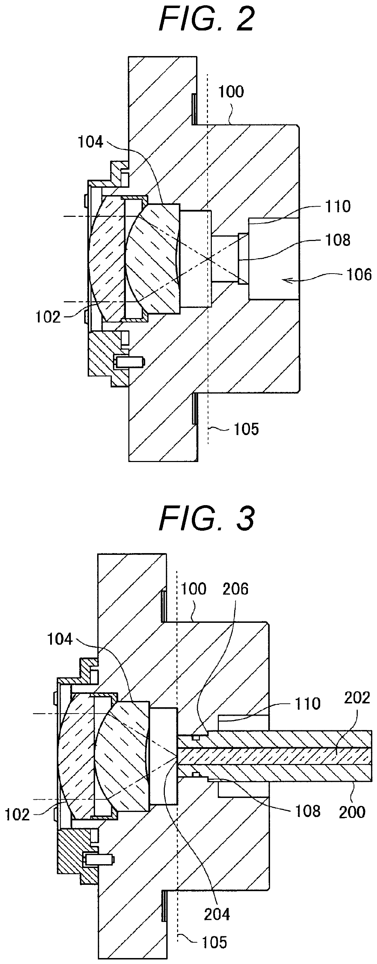

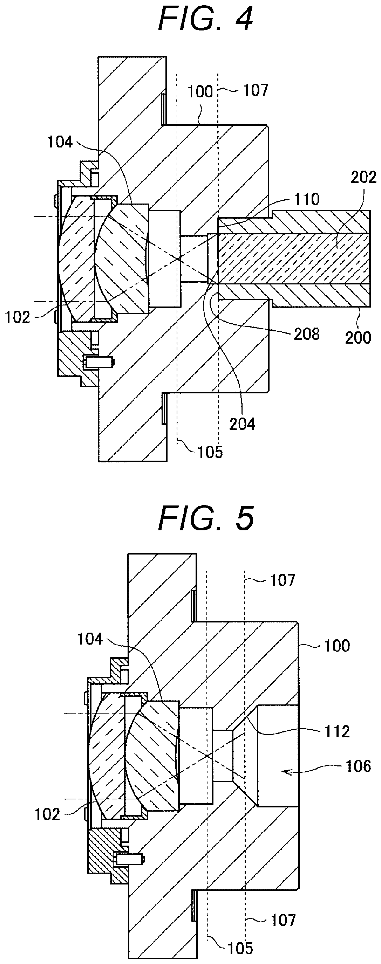

Fig. 2 is a cross-sectional view illustrating in detail the connection portion 100 of the light source device 1000. Furthermore, Figs. 3 and 4 are each a cross-sectional view illustrating in detail a state in which the connection portion 100 of the light source device 1000 is connected with the connection portion 200 of the light guide cable 2000. Figs. 2 to 4 also illustrate cross sections passing through the central axis of the connection portion 100 and the connection portion 200.

The connection portion 100 of the light source device 1000 includes a lens 102 and a lens 104 for collecting light emitted by the light source device 1000. Chain double-dashed lines illustrated in Figs. 2 to 4 illustrate how light emitted from the light source device 1000 is collected. As illustrated in Figs. 2 to 4, the light emitted from the light source device 1000 is formed as an image on a focal plane 105.

The connection portion 100 of the light source device 1000 is provided with an insertion portion 106 into which the connection portion 200 of the light guide cable 2000 is inserted. An inner surface of the insertion portion 106 has a stepped shape, and is provided with a first surface 108 and a second surface 110 that function as stoppers when the connection portion 200 of the light guide cable 2000 is inserted.

The light guide cable 2000 has a light guide portion 202 that guides light emitted from the light source device 1000. As illustrated in Figs. 3 and 4, the light emitted from the light source device 1000 enters from an entrance end face 204 of the light guide portion 202.

Figs. 3 and 4 illustrate states in which two types of light guide cables 200 that differ in thickness of the light guide portion 202 are each attached to the connection portion 100 of the light source device 1000. The light guide portion 202 of the light guide cable 2000 illustrated in Fig. 3 is thinner than the light guide portion 202 of the light guide cable 2000 illustrated in Fig. 4, and an outer diameter of a tip of the connection portion 200 is larger in Fig. 3 than in Fig. 4.

The tip of the connection portion 200 illustrated in Fig. 3 has a stepped outer shape, and is provided with a surface 206. As illustrated in Fig. 3, the surface 206 is in contact with the first surface 108 of the connection portion 100 of the light source device 1000.

Furthermore, in the example illustrated in Fig. 4, a surface 208 constituting the same surface with the entrance end face 204 is in contact with the second surface 110 of the connection portion 100 of the light source device 1000.

With the above configuration, when the connection portion 200 is attached to the connection portion 100, a position of the entrance end face 204 of the light guide cable 2000 on an optical axis is fixed at a different position in accordance with a diameter of the light guide portion 202 (diameter of the tip of the connection portion 200).

Then, with the above configuration, the position on the optical axis of the entrance end face 204 of the light guide portion 202 is arranged at a position where illumination light emitted by the light source device 1000 enters the light guide portion 202 most and uniformly.

In the example illustrated in Fig. 3, the position of the entrance end face 204 substantially coincides with a position of the focal plane 105. In a case where the light guide cable 2000 with a smaller diameter as illustrated in Fig. 3 is used, the connection portion 200 bumps into the connection portion 100 at a position where the focal plane 105 of illumination light and the entrance end face 204 of the light guide cable 2000 are coincident with each other in position on the optical axis. With this arrangement, the light guide cable 2000 is fixed at a position where illumination light enters most and uniformly.

Furthermore, in the example illustrated in Fig. 4, since the entrance end face 204 of the light guide portion 202 has a diameter larger than that in Fig. 3, the position of the entrance end face 204 is arranged at a position of a non-focal plane 107 located further to the right of the focal plane 105. In Fig. 4, light is formed as an image on the focal plane 105, and then light flux spreads on the right side of the focal plane 105. At the position of the non-focal plane 107 (that is, the position of the entrance end face 204), the luminous flux has a diameter that substantially coincides with the diameter of the entrance end face 204. In a case where the light guide cable 2000 with a larger diameter as illustrated in Fig. 4 is used, the connection portion 200 bumps into the connection portion 100 at a position where the non-focal plane 107 where illumination light enters most and uniformly and the entrance end face 204 are coincident with each other in position on the optical axis. With this arrangement, the light guide cable 2000 is fixed at a position where illumination light enters most and uniformly.

Thus, according to the examples illustrated in Figs. 3 and 4, illumination light emitted from the light source device 1000 can enter the light guide portion 202 most and uniformly in accordance with the diameter of the light guide portion 202. Note that, also in the example illustrated in Fig. 3, the position of the entrance end face 204 may be arranged further to the right of the focal plane 105 so that the diameter of the light flux at the position of the entrance end face 204 may coincide with the diameter of the entrance end face 204.

With the above configuration, even in a case where the diameter of the light flux at the focal plane 105 of illumination light is smaller than the diameter of the light guide portion 202 of the light guide cable 2000, such as a case where laser light is used as the light source, the position of the entrance end face 204 can be fixed at a position where illumination light enters most and uniformly regardless of the size of the diameter of the light guide portion 202.

Furthermore, owing to the stepped shape of the insertion portion 106 into which the light guide cable 2000 is inserted, even in a case of attachment and detachment or replacement between the light guide cables 2000 that differ in diameter of the light guide portion 202 as illustrated in Figs. 3 and 4, the entrance end face 204 can be fixed at a position where illumination light enters most and uniformly without any special adjustment. Note that, although Figs. 3 and 4 illustrate an example in which the connection portion 100 has a stepped shape having two steps and two surfaces (the first surface 108 and the second surface 110), more butting surfaces may be formed so as to support more types of light guide cables 2000.

### 3. Variations of present embodiment

### 3.1. Example in which insertion portion has tapered surface

Next, some variations of the present embodiment will be described on the basis of Figs. 5 to 11. Fig. 5 is a cross-sectional view illustrating in detail the connection portion 100 of the light source device 1000. The connection portion 100 illustrated in Fig. 5 has a configuration basically similar to that illustrated in Fig. 2, except that the insertion portion 106 of the connection portion 100 is provided with a tapered surface (mortar shape) 112.

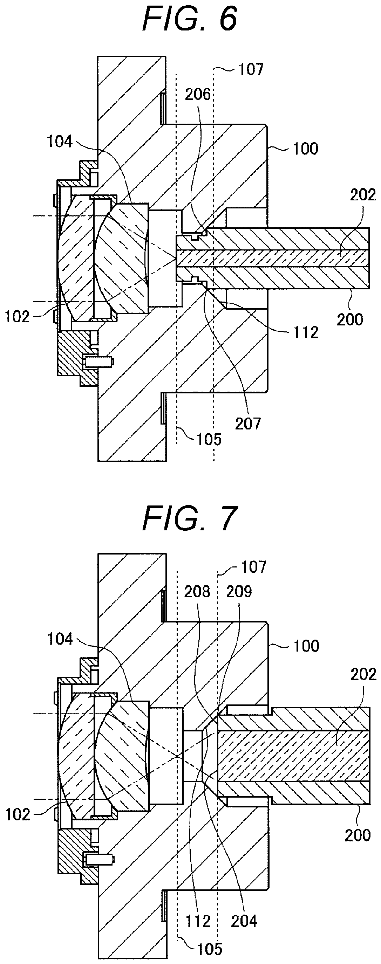

Figs. 6 and 7 are cross-sectional views each illustrating in detail a state in which the connection portion 100 of the light source device 1000 illustrated in Fig. 5 is connected with the connection portion 200 of the light guide cable 2000. Figs. 5 to 7 also illustrate cross sections passing through the central axis of the connection portion 100 and the connection portion 200.

The tapered surface 112 provided on the insertion portion 106 of the connection portion 100 functions as a stopper when the connection portion 200 of the light guide cable 2000 is inserted. Figs. 6 and 7 illustrate states in which two types of light guide cables 200 that differ in thickness of the light guide portion 202 are each attached to the connection portion 100 of the light source device 1000 illustrated in Fig. 5. The connection portion 200 illustrated in Fig. 6 has a configuration similar to the configuration of the connection portion 200 illustrated in Fig. 3, and the connection portion 200 illustrated in Fig. 7 has a configuration similar to the configuration of the connection portion 200 illustrated in Fig. 4. The light guide portion 202 of the light guide cable 2000 illustrated in Fig. 6 is thinner than the light guide portion 202 of the light guide cable 2000 illustrated in Fig. 7, and the outer diameter of the tip of the connection portion 200 is larger in Fig. 6 than in Fig. 7.

As illustrated in Fig. 6, when the connection portion 200 is attached to the connection portion 100, an outer edge 207 of the surface 206 provided on the connection portion 200 comes into contact with the tapered surface 112 of the connection portion 100.

Furthermore, in the example illustrated in Fig. 7, an outer edge 209 of the surface 208 constituting the same surface with the entrance end face 204 comes into contact with the tapered surface 112 of the connection portion 100.

With the above configuration, when the connection portion 200 is attached to the connection portion 100, the position of the entrance end face 204 of the light guide cable 2000 on the optical axis is fixed at a different position in accordance with the diameter of the light guide portion 202 (the diameter of the tip of the connection portion 200).

Then, as in Figs. 3 and 4, the position on the optical axis of the entrance end face 204 of the light guide portion 202 is arranged at a position where illumination light emitted by the light source device 1000 enters the light guide portion 202 most and uniformly. In the example illustrated in Fig. 6, the position of the entrance end face 204 substantially coincides with the position of the focal plane 105. In the example illustrated in Fig. 7, since the entrance end face 204 of the light guide portion 202 has a diameter larger than that in Fig. 6, the position of the entrance end face 204 is arranged at a position of a non-focal plane 107 located further to the right of the focal plane 105.

### 3.2. Example in which inner surface of insertion portion is stepped only in part of inner diameter

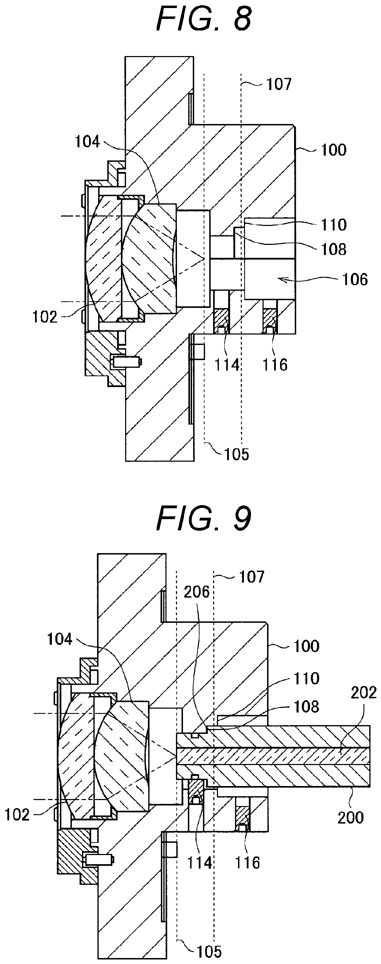

Fig. 8 is a cross-sectional view illustrating an example in which the inner surface of the insertion portion 106 of the connection portion 100 in the configuration illustrated in Fig. 2 is stepped only in a part of an inner diameter. In the configuration illustrated in Fig. 8, on the inner surface centered on the optical axis of the insertion portion 106, an area of 180° centered on the optical axis has a stepped shape similar to that in Fig. 2, and the remaining area of 180° has a screwing structure for fixing the connection portion 200. That is, only the area of 180° centered on the optical axis is stepped, and the remaining area of 180° is not stepped. As described above, even in a case where only a part of the inner diameter of the insertion portion 106 is stepped, the connection portion 200 can be positioned in an optical axis direction with respect to the connection portion 100.

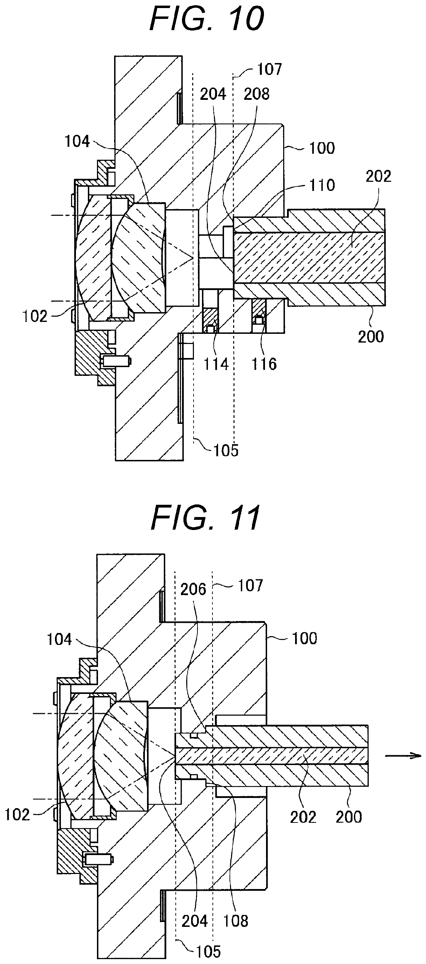

Figs. 9 and 10 are cross-sectional views each illustrating in detail a state in which the connection portion 100 of the light source device 1000 illustrated in Fig. 8 is connected with the connection portion 200 of the light guide cable 2000. Figs. 8 to 10 also illustrate cross sections passing through the central axis of the connection portion 100 and the connection portion 200.

Figs. 9 and 10 also illustrate states in which two types of light guide cables 200 that differ in thickness of the light guide portion 202 are each attached to the connection portion 100 of the light source device 1000 illustrated in Fig. 8. The connection portion 200 illustrated in Fig. 9 has a configuration similar to the configuration of the connection portion 200 illustrated in Fig. 3, and the connection portion 200 illustrated in Fig. 10 has a configuration similar to the configuration of the connection portion 200 illustrated in Fig. 4.

On the inner surface centered on the optical axis of the insertion portion 106 of the connection portion 100, an area of 180° centered on the optical axis has a stepped shape similar to that in Fig. 2, and in the example illustrated in Fig. 9, the surface 206 of the connection portion 200 comes into contact with the first surface 108 of the connection portion 100. Furthermore, in the example illustrated in Fig. 10, the surface 208 constituting the same surface with the entrance end face 204 comes into contact with the second surface 110 of the connection portion 100 of the light source device 1000. That is, the entrance end face 204 is positioned by the stepped shape in a similar manner to Figs. 3 and 4. Thus, as in Figs. 3 and 4, the position on the optical axis of the entrance end face 204 of the light guide portion 202 is arranged at a position where illumination light emitted by the light source device 1000 enters the light guide portion 202 most and uniformly.

Furthermore, the screwing structure includes two screws 114 and 116 that are screwed from an outer periphery of the connection portion 100 toward the optical axis. The screw 114 is tightened toward the connection portion 200 in a case where the light guide cable 2000 illustrated in Fig. 9 is attached to the light source device 1000. With this arrangement, the screw 114 presses the connection portion 200, thereby fixing the connection portion 200 to the connection portion 100.

Furthermore, the screw 116 is tightened toward the connection portion 200 in a case where the light guide cable 2000 illustrated in Fig. 10 is attached to the light source device 1000. With this arrangement, the screw 116 presses the connection portion 200, thereby fixing the connection portion 200 to the connection portion 100.

Thus, according to the configurations illustrated in Figs. 8 to 10, a space of the connection portion 100 can be effectively utilized by arranging the screwing structure in the area that is not stepped in an insertion portion 120. Furthermore, the screwing structure allows the connection portion 200 to be securely fixed to the connection portion 100.

### 3.3. Configuration example in which position of entrance end face can be changed by actuator

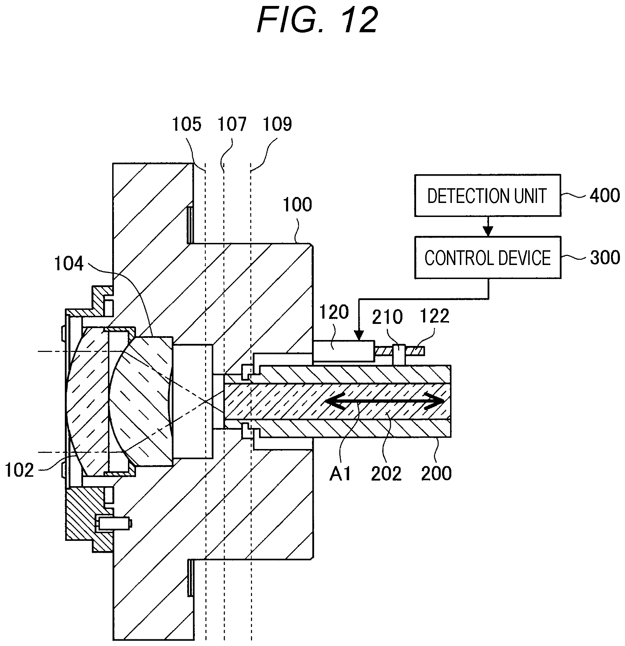

Next, on the basis of Figs. 11 and 12, a configuration in which positioning of the connection portion 200 in the optical axis direction with respect to the connection portion 100 can be changed by an actuator will be described.

The connection portion 100 illustrated in Figs. 11 and 12 has a configuration basically similar to that in Figs. 2 to 4. On the other hand, Figs. 11 and 12 illustrate states in which the light guide cables 2000 that are the same in outer diameter of the connection portion 200 but different in diameter of the light guide portion 202 are each attached. The light guide portion 202 illustrated in Fig. 11 has a diameter smaller than that of the light guide portion 202 illustrated in Fig. 12. The tip of the connection portion 200 has a shape similar to that of the connection portion 200 illustrated in Fig. 3.

As illustrated in Figs. 11 and 12, due to specifications of the light guide cables 2000, light guide cables 2000 that are the same in outer diameter and shape of the tip of the connection portion 200 may differ in diameter of the light guide portion 202 inside in some cases. The light guide cables 2000 illustrated in Figs. 11 and 12 are each provided with the surface 206. In the example illustrated in Fig. 11, the surface 206 is in contact with the first surface 108 of the connection portion 100 of the light source device 1000, as in Fig. 3. Thus, in Fig. 11, the position of the entrance end face 204 is fixed at a position where illumination light enters most and uniformly.

On the other hand, in the example illustrated in Fig. 12, the outer diameter of the connection portion 200 is the same, but the diameter of the light guide portion 202 is larger than that in Fig. 11, so the position of the entrance end face 204 is arranged not at the focal plane 105 as in Fig. 11 but at the position of the non-focal plane 107, where illumination light enters most and uniformly. However, the tip of the connection portion 200 illustrated in Figs. 11 and 12 has a shape similar to that in Fig. 3, and the position of the entrance end face 204 cannot be adjusted to the position of the non-focal plane 107 by mechanical contact. Then, in the example illustrated in Fig. 12, in a case where the position of the entrance end face 204 is fixed at the position of a focal plane 205, there arises a possibility that sufficient brightness of an observation target illuminated with light by the light guide cable 2000 cannot be secured.

Furthermore, for example, in a case of observing a screen obtained by imaging an observation target illuminated by the light guide cable 2000, it is desirable to maximize the brightness of the screen. Then, brightness of light guided through the light guide cable 2000 depends on the diameter of the light flux at the entrance end face 204, and it is therefore suitable to allow the entrance end face 204 to move to a position where illumination light enters most and uniformly.

For this reason, in the example illustrated in Fig. 12, a movement mechanism is provided to detect the brightness of the screen obtained by imaging an observation target illuminated by the light guide cable 2000, and move the position of the connection portion 200 from the position in Fig. 11 in a direction of an arrow A1. The movement mechanism includes an actuator (motor) 120 attached to the connection portion 100, a male screw (screw) 122 provided on a rotation axis of the actuator 120, and a moving member 210 that is attached to the connection portion 200 and has a female screw engaged with the male screw 122. The moving member 210 is attached to the connection portion 200 when the connection portion 200 is inserted into the connection portion 100.

Furthermore, in the example illustrated in Fig. 12, a control device 300 that controls the actuator 120 and a detection unit 400 that detects the brightness of the screen obtained by imaging an observation target illuminated by the light guide cable 2000 are provided. The control device 300 controls the actuator 120 on the basis of the brightness of the screen obtained from the detection unit 400. When the actuator 120 is driven, the male screw 122 provided on the rotation axis of the actuator 120 rotates, thereby causing the moving member 210 having the female screw engaged with the male screw 122 to move in the direction of the arrow A1. With this arrangement, by controlling the actuator 120 while monitoring the brightness of the screen by the detection unit 400, the entrance end face 204 of the connection portion 200 can be moved to a position where luminance of the screen becomes highest.

As described above, in the example illustrated in Fig. 12, in a case where the position of the entrance end face 204 is fixed at the position of the focal plane 105, there is a possibility that the brightness of the screen actually displayed is not sufficient. The detection unit 400 acquires the brightness of the screen, the actuator 120 is driven to move the position of the entrance end face 204 in the direction of the arrow A1, and the actuator 120 is stopped at a position where the brightness of the screen is maximized. Fig. 12 illustrates a state in which the position of the entrance end face 204 coincides with the position of a non-focal plane 109, and it is also possible to drive the actuator 120 to cause the position of the entrance end face 204 to coincide with a position of another non-focal plane 109. Thus, by providing a mechanism for moving the position of the connection portion 200, the position can be automatically adjusted to a position where illumination light enters most and uniformly.

### 4. Configuration example of medical system

### 4.1. Configuration example of endoscope system

The technology according to the present disclosure can be applied to a variety of products. For example, the technology according to the present disclosure may be applied to an endoscopic surgery system.

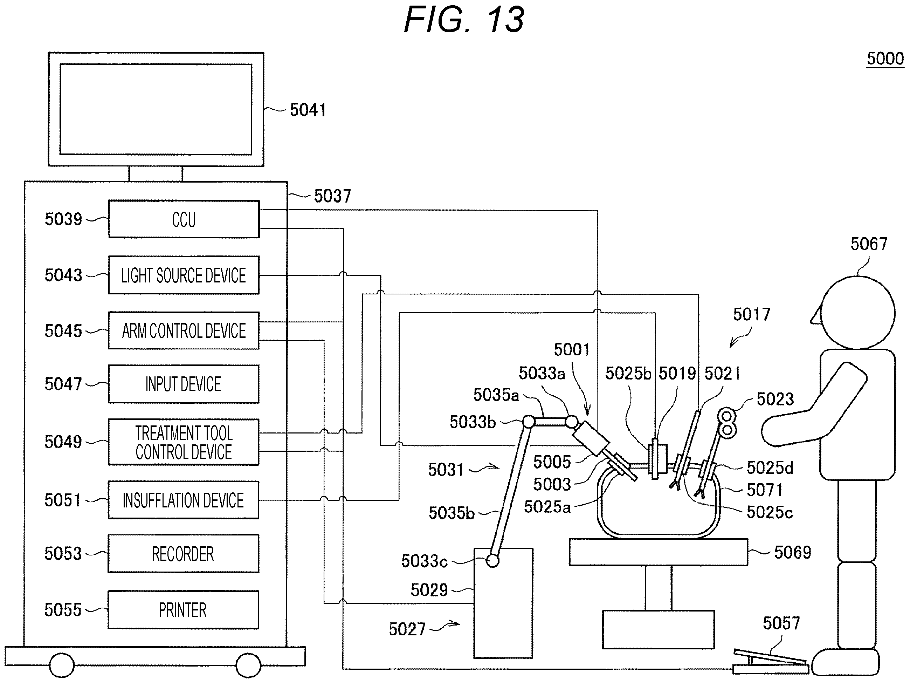

Fig. 13 is a diagram illustrating an example of a schematic configuration of an endoscopic surgery system 5000 to which the technology according to the present disclosure may be applied. Fig. 13 illustrates a situation in which an operator (doctor) 5067 is performing surgery on a patient 5071 on a patient bed 5069 using the endoscopic surgery system 5000. As illustrated, the endoscopic surgery system 5000 includes an endoscope 5001, other surgical tools 5017, a support arm device 5027 that supports the endoscope 5001, and a cart 5037 on which various devices for endoscopic surgery are mounted.

In endoscopic surgery, an abdominal wall is not cut and opened, but is pierced with a plurality of tubular hole-opening instruments called trocars 5025a to 5025d. Then, a lens barrel 5003 of the endoscope 5001 and the other surgical tools 5017 are inserted into a body cavity of the patient 5071 through the trocars 5025a to 5025d. In the illustrated example, an insufflation tube 5019, an energy treatment tool 5021, and forceps 5023 are inserted into the body cavity of the patient 5071 as the other surgical tools 5017. Furthermore, the energy treatment tool 5021 is used to perform incision and exfoliation of tissue, sealing of a blood vessel, or the like by using a high-frequency current or ultrasonic vibration. However, the illustrated surgical tools 5017 are merely an example, and various surgical tools generally used in endoscopic surgery, such as tweezers and a retractor, may be used as the surgical tools 5017.

An image of a surgical site in the body cavity of the patient 5071 captured by the endoscope 5001 is displayed on a display device 5041. The operator 5067 performs a procedure such as excision of an affected part, for example, using the energy treatment tool 5021 or the forceps 5023 while viewing the image of the surgical site displayed on the display device 5041 in real time. Note that, although not illustrated, the insufflation tube 5019, the energy treatment tool 5021, and the forceps 5023 are supported by the operator 5067, an assistant, or the like during the surgery.

### (Support arm device)

The support arm device 5027 includes an arm 5031 extending from a base portion 5029. In the illustrated example, the arm 5031 includes joints 5033a, 5033b, and 5033c, and links 5035a and 5035b, and is driven by control of an arm control device 5045. The arm 5031 supports the endoscope 5001 so as to control its position and orientation. With this arrangement, the position of the endoscope 5001 can be stably fixed.

### (Endoscope)

The endoscope 5001 includes the lens barrel 5003 whose predetermined length from its distal end is inserted into the body cavity of the patient 5071, and a camera head 5005 connected to a proximal end of the lens barrel 5003. In the illustrated example, the endoscope 5001 configured as a so-called rigid endoscope having the lens barrel 5003 that is rigid is illustrated. Alternatively, the endoscope 5001 may be configured as a so-called flexible endoscope having the lens barrel 5003 that is flexible.

The lens barrel 5003 is provided with, at the distal end thereof, an opening portion in which an objective lens is fitted. The endoscope 5001 is connected with a light source device 5043. Light generated by the light source device 5043 is guided to the distal end of the lens barrel by a light guide extending inside the lens barrel 5003, and is emitted through the objective lens toward an observation target in the body cavity of the patient 5071. Note that the endoscope 5001 may be a forward-viewing endoscope, an oblique-viewing endoscope, or a side-viewing endoscope.

The camera head 5005 is provided with an optical system and an imaging element inside thereof, and light reflected from the observation target (observation light) is collected on the imaging element by the optical system. The imaging element photoelectrically converts the observation light to generate an electric signal corresponding to the observation light, that is, an image signal corresponding to an observation image. The image signal is transmitted to a camera control unit (CCU) 5039 as raw data. Note that the camera head 5005 has a function of adjusting a magnification and a focal length by appropriately driving the optical system.

Note that the camera head 5005 may be provided with a plurality of imaging elements in order to support, for example, stereoscopic viewing (3D display). In this case, the lens barrel 5003 is provided with a plurality of relay optical systems inside thereof to guide observation light to each one of the plurality of imaging elements.

### (Various devices mounted on cart)

The CCU 5039 is constituted by a central processing unit (CPU), a graphics processing unit (GPU), and the like, and integrally controls operations of the endoscope 5001 and the display device 5041. Specifically, the CCU 5039 performs, on an image signal received from the camera head 5005, various types of image processing for displaying an image based on the image signal, such as development processing (demosaic processing), for example. The CCU 5039 provides the display device 5041 with the image signal on which the image processing has been performed. Furthermore, the CCU 5039 transmits a control signal to the camera head 5005 to control its driving. The control signal may contain information regarding imaging conditions such as the magnification and the focal length.

The CCU 5039 controls the display device 5041 to display an image based on the image signal on which image processing has been performed by the CCU 5039. In a case where, for example, the endoscope 5001 supports imaging with a high resolution such as 4K (3840 horizontal pixels x 2160 vertical pixels) or 8K (7680 horizontal pixels x 4320 vertical pixels), and/or in a case where the endoscope 5001 supports 3D display, a display device supporting high-resolution display and/or 3D display can be used accordingly as the display device 5041. In a case where imaging with a high resolution such as 4K or 8K is supported, a display device having a size of 55 inches or more can be used as the display device 5041 to provide more immersive feeling. Furthermore, a plurality of display devices 5041 having different resolutions and sizes may be provided in accordance with the intended use.

The light source device 5043 includes a light source such as a light emitting diode (LED), for example, and supplies the endoscope 5001 with emitted light at the time of imaging a surgical site.

The arm control device 5045 is constituted by a processor such as a CPU, and operates in accordance with a predetermined program to control driving of the arm 5031 of the support arm device 5027 in accordance with a predetermined control method.

An input device 5047 is an input interface to the endoscopic surgery system 5000. A user can input various types of information and input instructions to the endoscopic surgery system 5000 via the input device 5047. For example, the user may input, via the input device 5047, various types of information regarding surgery, such as physical information of a patient and information regarding a surgical procedure. Furthermore, for example, the user may input, via the input device 5047, an instruction to drive the arm 5031, an instruction to change imaging conditions (the type of emitted light, the magnification and focal length, and the like) of the endoscope 5001, an instruction to drive the energy treatment tool 5021, and the like.

The type of the input device 5047 is not limited, and various known input devices may be used as the input device 5047. As the input device 5047, for example, a mouse, a keyboard, a touch panel, a switch, a foot switch 5057, and/or a lever can be applied. In a case where a touch panel is used as the input device 5047, the touch panel may be provided on a display surface of the display device 5041.

Alternatively, the input device 5047 is a device worn by a user, such as a glasses-type wearable device or a head mounted display (HMD), for example, and various inputs are performed in accordance with a user's gesture or line-of-sight detected by these devices. Furthermore, the input device 5047 includes a camera capable of detecting a movement of a user, and various inputs are performed in accordance with a user's gesture or line-of-sight detected from a video captured by the camera. Moreover, the input device 5047 includes a microphone capable of collecting a user's voice, and various inputs are performed by speech via the microphone. As described above, the input device 5047 has a configuration in which various types of information can be input in a non-contact manner, and this allows, in particular, a user belonging to a clean area (for example, the operator 5067) to operate equipment belonging to an unclean area in a non-contact manner. Furthermore, the user can operate the equipment while holding a surgical tool in hand, and this improves convenience of the user.

A treatment tool control device 5049 controls driving of the energy treatment tool 5021 for cauterization or incision of tissue, sealing of a blood vessel, or the like. An insufflation device 5051 sends gas through the insufflation tube 5019 into the body cavity in order to inflate a body cavity of the patient 5071 for the purpose of securing a field of view of the endoscope 5001 and securing a working space for the operator. A recorder 5053 is a device that can record various types of information regarding surgery. A printer 5055 is a device that can print various types of information regarding surgery in various formats such as text, images, or graphs.

A particularly characteristic configuration of the endoscopic surgery system 5000 will be described below in more detail.

### (Support arm device)

The support arm device 5027 includes the base portion 5029 as a base, and the arm 5031 extending from the base portion 5029. In the illustrated example, the arm 5031 includes the plurality of joints 5033a, 5033b, and 5033c, and the plurality of links 5035a and 5035b coupled by the joint 5033b. However, Fig. 13 illustrates a configuration of the arm 5031 in a simplified manner for ease. In practice, the shapes, the numbers, and the arrangement of the joints 5033a to 5033c and the links 5035a and 5035b, the directions of rotation axes of the joints 5033a to 5033c, and the like can be appropriately set so that the arm 5031 has a desired degree of freedom. For example, the arm 5031 may suitably have a configuration that enables six or more degrees of freedom. With this arrangement, the endoscope 5001 can be freely moved within a movable range of the arm 5031, and the lens barrel 5003 of the endoscope 5001 can be inserted into the body cavity of the patient 5071 from a desired direction.

The joints 5033a to 5033c are provided with actuators, and the joints 5033a to 5033c have a configuration that enables rotation about a predetermined rotation axis by driving of the actuators. The arm control device 5045 controls the driving of the actuators, thereby controlling a rotation angle of each of the joints 5033a to 5033c, and controlling the driving of the arm 5031. With this arrangement, the position and orientation of the endoscope 5001 can be controlled. At this time, the arm control device 5045 can control the driving of the arm 5031 by various known control methods such as force control or position control.

For example, the position and orientation of the endoscope 5001 may be controlled by the operator 5067 performing an appropriate operation input via the input device 5047 (including the foot switch 5057), thereby causing the arm control device 5045 to appropriately control the driving of the arm 5031 in accordance with the operation input. With this control, the endoscope 5001 at a distal end of the arm 5031 can be moved from an optional position to an optional position, and then fixedly supported at the position after the movement. Note that the arm 5031 may be operated by a so-called master-slave method. In this case, the arm 5031 can be remotely controlled by a user via the input device 5047 installed at a location away from a surgery room.

Furthermore, in a case where the force control is applied, so-called power assist control may be performed in which the arm control device 5045 receives an external force from a user and drives the actuators of the corresponding joints 5033a to 5033c so that the arm 5031 moves smoothly in accordance with the external force. With this arrangement, when the user moves the arm 5031 while directly touching the arm 5031, the arm 5031 can be moved with a relatively light force. Thus, the endoscope 5001 can be moved more intuitively and with a simpler operation, and this improves convenience of the user.

Here, in general, the endoscope 5001 has been supported by a doctor called an endoscopist during endoscopic surgery. On the other hand, by using the support arm device 5027, the position of the endoscope 5001 can be fixed more securely without manual operation. This makes it possible to stably obtain an image of a surgical site and smoothly perform surgery.

Note that the arm control device 5045 is not necessarily provided at the cart 5037. Furthermore, the arm control device 5045 is not necessarily one device. For example, the arm control device 5045 may be provided one for each of the joints 5033a to 5033c of the arm 5031 of the support arm device 5027, and a plurality of the arm control devices 5045 may cooperate with one another to control the driving of the arm 5031.

### (Light source device)

The light source device 5043 supplies the endoscope 5001 with emitted light at the time of imaging a surgical site. The light source device 5043 is constituted by a white light source including, for example, an LED, a laser light source, or a combination thereof. At this time, in a case where the white light source is constituted by a combination of RGB laser light sources, an output intensity and output timing of each color (each wavelength) can be controlled with high precision, and this enables white balance adjustment of a captured image at the light source device 5043. Furthermore, in this case, an image for each of R, G, and B can be captured in a time-division manner by emitting laser light from each of the RGB laser light sources to an observation target in a time-division manner, and controlling driving of the imaging element of the camera head 5005 in synchronization with the emission timing. According to this method, a color image can be obtained without providing a color filter in the imaging element.

Furthermore, driving of the light source device 5043 may be controlled so that the intensity of light to be output changes at a predetermined time interval. By controlling the driving of the imaging element of the camera head 5005 in synchronization with the timing of the change in the light intensity, acquiring images in a time-division manner, and generating a composite image from the images, a high dynamic range image without so-called blocked up shadows or blown out highlights can be generated.

Furthermore, the light source device 5043 may have a configuration in which light can be supplied in a predetermined wavelength band that can be used for special light observation. In special light observation, for example, by utilizing wavelength dependence of light absorption in body tissue, so-called narrow band imaging is performed in which a predetermined tissue such as a blood vessel in a mucosal surface layer is imaged with high contrast by emitting light in a band narrower than that of light emitted during normal observation (that is, white light). Alternatively, in special light observation, fluorescence observation may be performed in which an image is obtained by fluorescence generated by emitting excitation light. In fluorescence observation, for example, excitation light is emitted to body tissue and fluorescence from the body tissue is observed (autofluorescence observation), or a fluorescent image is obtained by locally injecting a reagent such as indocyanine green (ICG) into body tissue and emitting excitation light corresponding to a fluorescence wavelength of the reagent to the body tissue. The light source device 5043 may have a configuration in which narrow-band light and/or excitation light that can be used for such special light observation can be supplied.

### (Camera head and CCU)

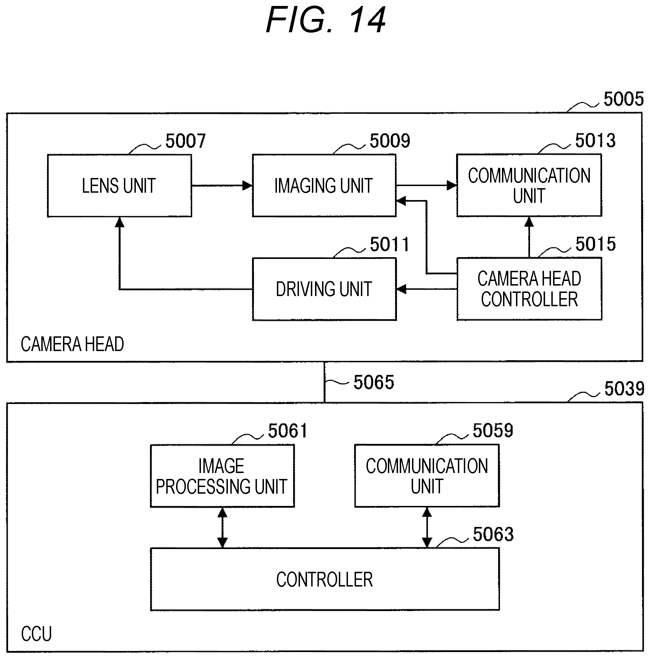

Functions of the camera head 5005 of the endoscope 5001 and the CCU 5039 will be described in more detail with reference to Fig. 14. Fig. 14 is a block diagram illustrating an example of a functional configuration of the camera head 5005 and the CCU 5039 illustrated in Fig. 13.

Referring to Fig. 14, the camera head 5005 has functions including a lens unit 5007, an imaging unit 5009, a driving unit 5011, a communication unit 5013, and a camera head controller 5015. Furthermore, the CCU 5039 has functions including a communication unit 5059, an image processing unit 5061, and a controller 5063. The camera head 5005 and the CCU 5039 are connected by a transmission cable 5065 to allow two-way communication.

First, the functional configuration of the camera head 5005 will be described. The lens unit 5007 is an optical system provided at a connection with the lens barrel 5003. Observation light taken in from the distal end of the lens barrel 5003 is guided to the camera head 5005 and enters the lens unit 5007. The lens unit 5007 is constituted by a combination of a plurality of lenses including a zoom lens and a focus lens. Optical characteristics of the lens unit 5007 are adjusted so that observation light may be collected on a light receiving surface of an imaging element of the imaging unit 5009. Furthermore, the zoom lens and the focus lens have a configuration in which their positions can be moved on an optical axis for adjustment of a magnification and a focus of a captured image.

The imaging unit 5009 includes the imaging element, and is arranged at a stage subsequent to the lens unit 5007. Observation light that has passed through the lens unit 5007 is collected on the light receiving surface of the imaging element, and an image signal corresponding to an observation image is generated by photoelectric conversion. The image signal generated by the imaging unit 5009 is provided to the communication unit 5013.

As the imaging element included in the imaging unit 5009, for example, a complementary metal oxide semiconductor (CMOS) type image sensor that has a Bayer array and can capture a color image is used. Note that, as the imaging element, an imaging element capable of capturing a high-resolution image of, for example, 4K or more may be used. An image of a surgical site can be obtained with a high resolution, and this allows the operator 5067 to grasp the state of the surgical site in more detail, and proceed with surgery more smoothly.

Furthermore, the imaging element included in the imaging unit 5009 has a configuration including a pair of imaging elements, one for acquiring a right-eye image signal and the other for acquiring a left-eye image signal supporting 3D display. The 3D display allows the operator 5067 to grasp the depth of living tissue in the surgical site more accurately. Note that, in a case where the imaging unit 5009 has a multi-plate type configuration, a plurality of the lens units 5007 is provided for the corresponding imaging elements.

Furthermore, the imaging unit 5009 is not necessarily provided in the camera head 5005. For example, the imaging unit 5009 may be provided inside the lens barrel 5003 just behind the objective lens.

The driving unit 5011 is constituted by an actuator, and the camera head controller 5015 controls the zoom lens and the focus lens of the lens unit 5007 to move by a predetermined distance along the optical axis. With this arrangement, the magnification and the focus of an image captured by the imaging unit 5009 can be appropriately adjusted.

The communication unit 5013 is constituted by a communication device for transmitting and receiving various types of information to and from the CCU 5039. The communication unit 5013 transmits an image signal obtained from the imaging unit 5009 as raw data to the CCU 5039 via the transmission cable 5065. At this time, it is preferable that the image signal be transmitted by optical communication in order to display a captured image of a surgical site with a low latency. This is because, during surgery, the operator 5067 performs surgery while observing a state of an affected part from a captured image, and it is required that a moving image of the surgical site be displayed in real time as much as possible for safer and more reliable surgery. In a case where optical communication is performed, the communication unit 5013 is provided with a photoelectric conversion module that converts an electric signal into an optical signal. An image signal is converted into an optical signal by the photoelectric conversion module, and then transmitted to the CCU 5039 via the transmission cable 5065.

Furthermore, the communication unit 5013 receives a control signal for controlling driving of the camera head 5005 from the CCU 5039. The control signal contains information regarding imaging conditions such as information for specifying a frame rate of a captured image, information for specifying an exposure value at the time of imaging, and/or information for specifying a magnification and focus of the captured image. The communication unit 5013 provides the received control signal to the camera head controller 5015. Note that the control signal from the CCU 5039 may also be transmitted by optical communication. In this case, the communication unit 5013 is provided with a photoelectric conversion module that converts an optical signal into an electric signal. The control signal is converted into an electric signal by the photoelectric conversion module, and then provided to the camera head controller 5015.

Note that the above-described imaging conditions such as the frame rate, the exposure value, the magnification, and the focus are automatically set by the controller 5063 of the CCU 5039 on the basis of an acquired image signal. That is, the endoscope 5001 has a so-called auto exposure (AE) function, an auto focus (AF) function, and an auto white balance (AWB) function.

The camera head controller 5015 controls the driving of the camera head 5005 on the basis of the control signal from the CCU 5039 received via the communication unit 5013. For example, the camera head controller 5015 controls driving of the imaging element of the imaging unit 5009 on the basis of information for specifying a frame rate of a captured image and/or information for specifying exposure at the time of imaging. Furthermore, for example, the camera head controller 5015 appropriately moves the zoom lens and the focus lens of the lens unit 5007 via the driving unit 5011 on the basis of information for specifying a magnification and a focus of a captured image. The camera head controller 5015 may further include a function of storing information for recognizing the lens barrel 5003 and the camera head 5005.

Note that, by arranging the configurations of the lens unit 5007, the imaging unit 5009, and the like in a hermetically sealed structure having high airtightness and waterproofness, the camera head 5005 can have resistance to autoclave sterilization.

Next, the functional configuration of the CCU 5039 will be described. The communication unit 5059 is constituted by a communication device for transmitting and receiving various types of information to and from the camera head 5005. The communication unit 5059 receives an image signal transmitted from the camera head 5005 via the transmission cable 5065. At this time, as described above, the image signal can be suitably transmitted by optical communication. In this case, to support optical communication, the communication unit 5059 is provided with a photoelectric conversion module that converts an optical signal into an electric signal. The communication unit 5059 provides the image processing unit 5061 with the image signal converted into an electric signal.

Furthermore, the communication unit 5059 transmits a control signal for controlling the driving of the camera head 5005 to the camera head 5005. The control signal may also be transmitted by optical communication.

The image processing unit 5061 performs various types of image processing on an image signal that is raw data transmitted from the camera head 5005. Examples of the image processing include various types of known signal processing such as development processing, high image quality processing (such as band emphasis processing, super-resolution processing, noise reduction (NR) processing, and/or camera shake correction processing), and/or enlargement processing (electronic zoom processing). Furthermore, the image processing unit 5061 performs demodulation processing on the image signal for performing AE, AF, and AWB.

The image processing unit 5061 is constituted by a processor such as a CPU or a GPU, and the image processing and demodulation processing described above can be performed by the processor operating in accordance with a predetermined program. Note that, in a case where the image processing unit 5061 is constituted by a plurality of GPUs, the image processing unit 5061 appropriately divides information related to the image signal, and image processing is performed in parallel by the plurality of GPUs.

The controller 5063 performs various controls regarding capturing of an image of a surgical site by the endoscope 5001 and display of the captured image. For example, the controller 5063 generates a control signal for controlling the driving of the camera head 5005. At this time, in a case where imaging conditions have been input by a user, the controller 5063 generates a control signal on the basis of the input by the user. Alternatively, in a case where the endoscope 5001 has an AE function, an AF function, and an AWB function, the controller 5063 appropriately calculates an optimal exposure value, focal length, and white balance in accordance with a result of demodulation processing performed by the image processing unit 5061, and generates a control signal.

Furthermore, the controller 5063 causes the display device 5041 to display an image of a surgical site on the basis of an image signal on which the image processing unit 5061 has performed image processing. At this time, the controller 5063 uses various image recognition technologies to recognize various objects in the image of the surgical site. For example, the controller 5063 can recognize a surgical tool such as forceps, a specific living body site, bleeding, mist at the time of using the energy treatment tool 5021, and the like by detecting a shape, color, and the like of an edge of an object in the image of the surgical site. When the image of the surgical site is displayed on the display device 5041, the controller 5063 superimposes various types of surgery support information upon the image of the surgical site using results of the recognition. The surgery support information is superimposed and presented to the operator 5067, and this allows surgery to be performed more safely and reliably.

The transmission cable 5065 connecting the camera head 5005 and the CCU 5039 is an electric signal cable that supports electric signal communication, an optical fiber cable that supports optical communication, or a composite cable thereof.

Here, in the illustrated example, wired communication is performed using the transmission cable 5065, but wireless communication may be performed between the camera head 5005 and the CCU 5039. In a case where wireless communication is performed between the two, the transmission cable 5065 does not need to be laid in the surgery room. This may resolve a situation in which movement of medical staff in the surgery room is hindered by the transmission cable 5065.

The example of the endoscopic surgery system 5000 to which the technology according to the present disclosure can be applied has been described above. Note that, although the endoscopic surgery system 5000 has been described as an example here, systems to which the technology according to the present disclosure can be applied are not limited to such an example. For example, the technology according to the present disclosure may be applied to an inspection flexible endoscope system or a microscopic surgery system.

The technology according to the present disclosure can be suitably applied to a connection structure between the light guide that guides light generated by the light source device 5043 to the distal end of the lens barrel 5003 and the light source device 5043, among the configurations described above. With this arrangement, it is possible to illuminate an observation target with uniform light with high luminance.

### 8.2. Configuration example of microscope system

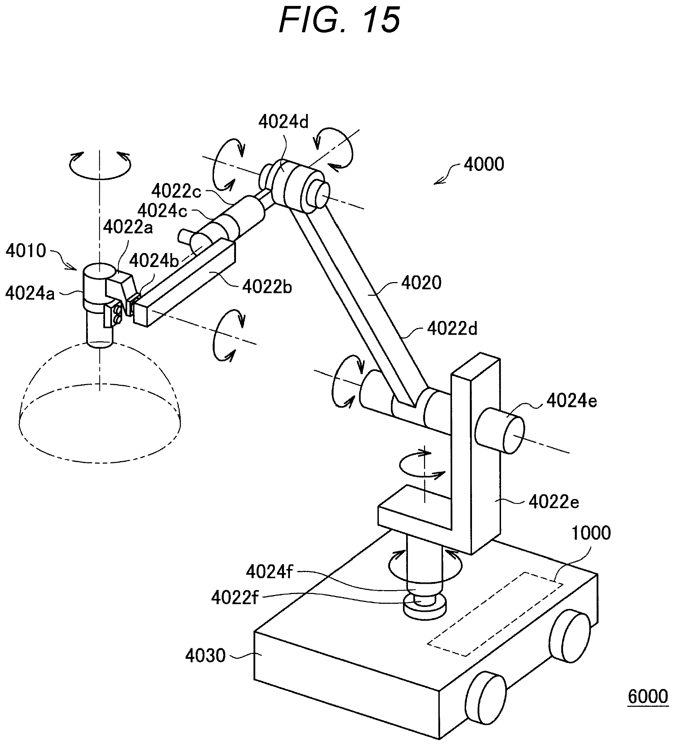

Fig. 15 is a diagram illustrating an example of a schematic configuration of a microscopic surgery system 6000 to which the light source device 1000 according to the present disclosure can be applied. Referring to Fig. 15, the microscopic surgery system 6000 includes a microscope device 4000 and the light source device 1000.

The microscope device 4000 includes a microscope unit 4010 for zooming in and observing an observation target (patient's surgical site), an arm 4020 having a distal end that supports the microscope unit 4010, and a base portion 4030 that supports a proximal end of the arm 4020.

The microscope unit 4010 is an electronic imaging microscope unit (so-called video microscope unit) that electronically captures an image by an imaging unit. Light from the observation target (hereinafter, also referred to as observation light) enters the imaging unit inside the microscope unit 4010.

The imaging unit includes an optical system that collects observation light and an imaging element that receives the observation light collected by the optical system. The optical system includes a combination of a plurality of lenses including a zoom lens and a focus lens, and the optical characteristics thereof are adjusted so that observation light is formed as an image on a light receiving surface of the imaging element. The imaging element receives observation light and performs photoelectric conversion to generate a signal corresponding to the observation light, that is, an image signal corresponding to an observation image. The imaging element used is, for example, an imaging element that has a Bayer array and can capture a color image. The imaging element may be any of various known imaging elements such as a complementary metal oxide semiconductor (CMOS) image sensor or a charge coupled device (CCD) image sensor.

The arm 4020 has a configuration in which a plurality of links (first link 4022a to sixth link 4022f) is rotatably coupled to each other by a plurality of joints (first joint 4024a to sixth joint 4024f). Each joint is rotatable about a rotation axis indicated by a long dashed short dashed line.

Note that the number and shape (length) of the links, and the number, arrangement positions, direction of the rotation axis, and the like of the joints that constitute the illustrated arm 4020 may be appropriately designed so that a desired degree of freedom can be obtained. Furthermore, each of the first joint 4024a to the sixth joint 4024f may be provided with an actuator having a drive mechanism such as a motor, an encoder that detects a rotation angle of the corresponding joint, and the like. Then, by appropriately controlling driving of each actuator provided in the first joint 4024a to the sixth joint 4024f, an orientation of the arm 4020, that is, a position and orientation of a microscope unit 4000 can be controlled.

The light source device 1000 is built in the base portion 4030, for example. A light guide 500 connected to the light source device 1000 is guided to the microscope unit 4010 through the inside or outside of the first link 4022a to the sixth link 4022f. An observation target is illuminated with light from a distal end of the light guide 500 guided to the microscope unit 4010, and this increases luminance of the observation target and allows the observation target to be clearly imaged when the imaging unit inside the microscope unit 4010 images the observation target (affected part) of a patient.

As described above, according to the present embodiment, regardless of the size of diameter of the light guide portion 202, an entrance end face 202 of the light guide cable 2000 can be fixed at a position where illumination light enters most and uniformly, without any special adjustment. Thus, for example, even in a case where the size of light flux at a focal plane of illumination light is smaller than the diameter of the light guide portion 202 of the light guide cable 2000, the entrance end face 202 can be arranged at a position where illumination light enters most and uniformly. Thus, it is possible to provide brighter illumination with an endoscope and a surgical microscope suitable for the size of the surgical space being used, without lowering the amount and uniformity of illumination light that can enter.

The preferred embodiment of the present disclosure has been described above in detail with reference to the accompanying drawings, but the technical scope of the present disclosure is not limited to such an example. It is obvious that a person having ordinary knowledge in the technical field of the present disclosure can come up with various changes and modifications within the scope of the technical idea described in the claims, and such various changes and modifications are naturally understood to belong to the technical scope of the present disclosure.

For example, although a medical light source device has been described as an example in the above embodiment, the present technology is not limited to such an example. The present embodiment can be applied to a wide variety of general-purpose light source devices such as industrial light source devices.

Furthermore, the effects described in the present specification are merely illustrative or exemplary, and are not restrictive. That is, the technology according to the present disclosure can exhibit other effects that are obvious to those skilled in the art from the description in the present specification, in addition to or instead of the above effects.

Note that configurations as described below also belong to the technical scope of the present disclosure.
(1) A medical system including:
   medical equipment that includes an imaging unit that images an observation target; and
   a light source device that emits light for illuminating the observation target and illuminates the observation target with the light via a light guide cable, in which
   an entrance end face of the light guide cable is set to a different position with respect to a focal plane of light emitted from the light source device in accordance with a diameter of a light guide portion of the light guide cable.
(2) A connection structure between a light source device and a light guide cable, in which
   an entrance end face of the light guide cable is set to a different position with respect to a focal plane of light emitted from the light source device in accordance with a diameter of a light guide portion of the light guide cable.
(3) The connection structure according to (2), in which each one of a plurality of the light guide cables that differ in diameter of the light guide portion is connected to the light source device.
(4) The connection structure according to (2) or (3), in which
   the light source device has an insertion portion into which a tip of the light guide cable is inserted, and
   the light guide cable is positioned with respect to the insertion portion at a different position in an optical axis direction in accordance with an inner diameter of the insertion portion.
(5) The connection structure according to (4), in which
   the insertion portion has a stepped shape in which a plurality of contact surfaces is arranged at different positions in the optical axis direction in accordance with a plurality of the inner diameters of the insertion portion, and
   the light guide cable is positioned in the optical axis direction with respect to the insertion portion by contact of the light guide cable with any of the contact surfaces, the tip of the light guide cable varying in diameter in accordance with the diameter of the light guide portion.
(6) The connection structure according to (5), in which the stepped structure is provided in a predetermined range on a circumference centered on an optical axis.
(7) The connection structure according to (6), in which a screwing structure that fixes the light guide cable to the insertion portion by tightening of a screw is provided in an area where the stepped structure is not provided, other than the predetermined range.
(8) The connection structure according to (4), in which
   the insertion portion has a tapered surface that spreads toward an insertion opening, and
   the light guide cable is positioned with respect to the insertion portion at a different position in the optical axis direction in accordance with the inner diameter of the insertion portion by contact of the light guide cable with the tapered surface, the tip of the light guide cable varying in diameter in accordance with the diameter of the light guide portion.
(9) The connection structure according to (2), in which
   the light source device has an insertion portion into which a tip of the light guide cable is inserted, and
   includes an actuator that changes a position of the light guide cable in an optical axis direction with respect to the insertion portion.
(10) The connection structure according to (9), further including
   a detection unit that detects brightness of an observation target illuminated with light by the light guide cable, in which
   the actuator changes the position of the light guide cable in the optical axis direction with respect to the insertion portion on the basis of the brightness of the observation target.
(11) The connection structure according to any one of (2) to (10), in which the light source device and the light guide cable are used for medical purposes.
(12) A connection method of connecting a light source device and a light guide cable, the connection method including
   positioning a light entrance end face of the light guide cable at a different position with respect to a focal plane of light emitted from the light source device in accordance with a diameter of a light guide portion of the light guide cable.

### REFERENCE SIGNS LIST

- 106: Insertion portion
- 108: First surface
- 110: Second surface
- 112: Tapered surface
- 114, 116: Screw
- 120: Actuator
- 202: Light guide portion
- 204: Entrance end face
- 205: Focal plane
- 400: Detection unit
- 1000: Light source device
- 2000: Light guide cable

## Claims

1. A medical system comprising:
medical equipment that includes an imaging unit that images an observation target; and
a light source device that emits light for illuminating the observation target and illuminates the observation target with the light via a light guide cable, wherein
an entrance end face of the light guide cable is set to a different position with respect to a focal plane of light emitted from the light source device in accordance with a diameter of a light guide portion of the light guide cable.

2. A connection structure between a light source device and a light guide cable, wherein
an entrance end face of the light guide cable is set to a different position with respect to a focal plane of light emitted from the light source device in accordance with a diameter of a light guide portion of the light guide cable.

3. The connection structure according to claim 2, wherein each one of a plurality of the light guide cables that differ in diameter of the light guide portion is connected to the light source device.

4. The connection structure according to claim 2, wherein
the light source device has an insertion portion into which a tip of the light guide cable is inserted, and
the light guide cable is positioned with respect to the insertion portion at a different position in an optical axis direction in accordance with an inner diameter of the insertion portion.

5. The connection structure according to claim 4, wherein
the insertion portion has a stepped shape in which a plurality of contact surfaces is arranged at different positions in the optical axis direction in accordance with a plurality of the inner diameters of the insertion portion, and
the light guide cable is positioned in the optical axis direction with respect to the insertion portion by contact of the light guide cable with any of the contact surfaces, the tip of the light guide cable varying in diameter in accordance with the diameter of the light guide portion.

6. The connection structure according to claim 5, wherein the stepped structure is provided in a predetermined range on a circumference centered on an optical axis.

7. The connection structure according to claim 6, wherein a screwing structure that fixes the light guide cable to the insertion portion by tightening of a screw is provided in an area where the stepped structure is not provided, other than the predetermined range.

8. The connection structure according to claim 4, wherein
the insertion portion has a tapered surface that spreads toward an insertion opening, and
the light guide cable is positioned with respect to the insertion portion at a different position in the optical axis direction in accordance with the inner diameter of the insertion portion by contact of the light guide cable with the tapered surface, the tip of the light guide cable varying in diameter in accordance with the diameter of the light guide portion.

9. The connection structure according to claim 2, wherein
the light source device has an insertion portion into which a tip of the light guide cable is inserted, and
includes an actuator that changes a position of the light guide cable in an optical axis direction with respect to the insertion portion.

10. The connection structure according to claim 9, further comprising
a detection unit that detects brightness of an observation target illuminated with light by the light guide cable, wherein
the actuator changes the position of the light guide cable in the optical axis direction with respect to the insertion portion on a basis of the brightness of the observation target.

11. The connection structure according to claim 2, wherein the light source device and the light guide cable are used for medical purposes.

12. A connection method of connecting a light source device and a light guide cable, the connection method comprising
positioning a light entrance end face of the light guide cable at a different position with respect to a focal plane of light emitted from the light source device in accordance with a diameter of a light guide portion of the light guide cable.
